(19) Europäisches Patentamt
European Patent Office
Office européen des brevets

(11) EP 4 458 362 A1

# (12) EUROPEAN PATENT APPLICATION
published in accordance with Art. 153(4) EPC

(43) Date of publication:
**06.11.2024 Bulletin 2024/45**

(21) Application number: **21969813.1**

(22) Date of filing: **31.12.2021**

(51) International Patent Classification (IPC):
***A61K 31/724*** *(2006.01)* ***C12N 9/72*** *(2006.01)*
***A61K 31/365*** *(2006.01)* ***A61K 9/08*** *(2006.01)*
***A61K 9/16*** *(2006.01)* ***A61K 9/19*** *(2006.01)*
***A61K 9/28*** *(2006.01)* ***A61K 9/48*** *(2006.01)*
***A61K 9/00*** *(2006.01)* ***A61P 7/02*** *(2006.01)*
***A61P 9/10*** *(2006.01)* ***A61P 43/00*** *(2006.01)*

(52) Cooperative Patent Classification (CPC):
**A61K 9/00; A61K 9/08; A61K 9/16; A61K 9/19; A61K 9/28; A61K 9/48; A61K 31/365; A61K 31/724; A61P 7/02; A61P 9/10; A61P 43/00; C12N 9/6462**

(86) International application number:
**PCT/CN2021/143944**

(87) International publication number:
**WO 2023/123468 (06.07.2023 Gazette 2023/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Hangzhou Adamerck Pharmlabs Inc.**
**Hangzhou, Zhejiang 311112 (CN)**

(72) Inventor: **QI, Youmao**
**Hangzhou, Zhejiang 311112 (CN)**

(74) Representative: **Mewburn Ellis LLP**
**Aurora Building**
**Counterslip**
**Bristol BS1 6BX (GB)**

## (54) CARDIOVASCULAR AND CEREBROVASCULAR DRUG AND USE THEREOF

(57) Provided in the present invention are a cardiovascular and cerebrovascular drug and the use thereof. Specifically, the compound of the present invention has a structure as represented by formula (I), wherein each group is defined as described in the description. Further disclosed in the present invention is the use of the compound in treating thrombotic diseases, resisting inflammation and treating nerve injury.

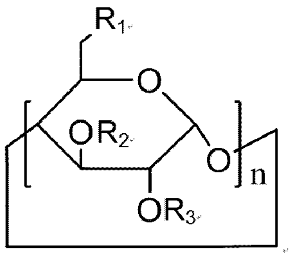

(I)

EP 4 458 362 A1

## Description

### Technical field

The present invention belongs to pharmaceutical field, more specifically relates to a cardiovascular and cerebrovascular drug and use thereof.

### Background

The incidence of cardiovascular disease in China is continuously increasing, with an estimated 330 million current patients. The incidence of stroke is still on the rise in China, with the number of new cases accounting for about 40% of the world's total, the number of death accounting for about 30% of the world's total, and the number of stroke patients surviving with the disease as high as 13 million, making China the country with the highest lifetime risk of stroke and the heaviest disease burden. It is estimated that the incidence of coronary heart disease in China will continue to rise over the next 10 years, and the incidence of acute myocardial infarction will also climb dramatically, and it is expected that after 2030 there will probably be more than 20 million patients with acute myocardial infarction in China. Acute pulmonary embolism is the third most common acute cardiovascular syndrome worldwide after myocardial infarction and stroke. The annual incidence rate of pulmonary embolism is (39-115) per 100,000 people, and the incidence has been on the rise in recent years.

When thrombotic diseases occur, surgical removal of the thrombus, thrombolysis, and antithrombotic therapy are mainly used clinically. Commonly used thrombolytic drugs include urokinase, streptokinase, alteplase, and reteplase, etc.. Commonly used antithrombotic drugs include antiplatelet agents and anticoagulants. Although thrombolytic drugs and oral antithrombotic drugs have good preventative and therapeutic effects, their disadvantage is that they both have a risk of bleeding, thus it is necessary to closely monitor the coagulation mechanism during their use.

Since vascular occlusion of the brain occurs during a stroke and the blood supply is terminated, causing damages to neuronal cells, clinical treatment of the damaged neuronal cells is required.

Edaravone is a drug developed and marketed in Japan for the treatment of acute attacks of cerebral infarction and improvement of neurological symptoms, daily living activities and dysfunctions caused by acute cerebral infarction, which has been approved for marketing in China but has not yet been recognized by mainstream countries such as Europe and the United States. Edaravone composition (edaravone camphenol) obtained partially positive results in the phase III clinical study and has been approved for marketing in China, but not approved and guidelines recommended by the U.S. FDA due to the inconsistency between its clinical protocol design and the current recognized clinical research guidelines for neuroprotective agents abroad.

The Chinese guidelines for the diagnosis and treatment of ischemic stroke only recommend butylphthalide as a class II drug to improve cerebral microcirculation, and there is no recommended neuroprotective agents in foreign clinical guidelines. It is fair to say that the field of neuroprotective agents recognized in mainstream medicine worldwide remains empty.

Therefore, the inventor envisions that the development of a novel therapeutic drug for stroke neurological damage, which can effectively perform thrombolytic therapy on thrombotic diseases while also play a role in protecting the cerebral nerves. This will fill the gaps in the field of neuroprotective agents and meet the unmet clinical needs in the field of cardiovascular and cerebrovascular diseases, with great clinical significance and practical value.

### SUMMARY OF THE INVENTION

The purpose of the present invention is to provide a cardiovascular and cerebrovascular drug and the use thereof.

In the first aspect of the present invention, provided is a use of a compound of formula I or a pharmaceutically acceptable salt thereof in the preparation of a pharmaceutical composition or formulation, the pharmaceutical composition or formulation comprises the compound of formula I as an active ingredient, and the pharmaceutical composition or formulation is used for:

(a) prevention and/or treatment of thrombosis-related diseases;
(b) anti-inflammatory treatment; and/or
(c) treatment of neuronal damage caused by cerebral infarction, traumatic brain injury, cerebral hemorrhage, or brain tumor surgery;

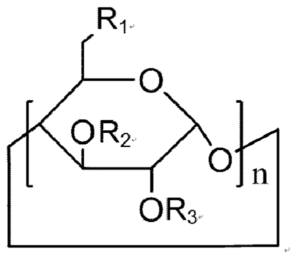

(I)

wherein,

$R_1$ is selected from the group consisting of: hydroxyl, C1-C6 alkoxy, halogen, substituted or unsubstituted -O-C1-C6 alkyl, and

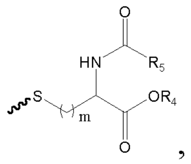,

wherein the substituted refers to substitution with sulfonic acid group or hydroxyl;
wherein, m is an integer selected from 1 to 6;
the carbon atom in

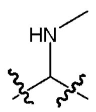 of 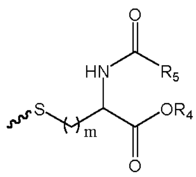

is chiral carbon atom, and the chirality is selected form the group consisting of:

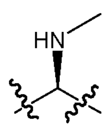 and 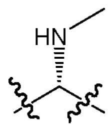;

$R_4$ is a metal ion selected from the group consisting of: $Na^+$, $K^+$, $Li^+$, and $Cs^+$;
$R_5$ is C1-C6 alkyl, C3-C8 cycloalkyl, C2-C6 alkenyl, C2-C6 alkynyl, aryl or heteroaryl;
$R_2$ and $R_3$ are each independently selected from: H, C1-C6 alkyl, C2-C6 alkenyl, C2-C6 alkynyl, C3-C8 cycloalkyl, C2-C6 hydroxyalkyl, and -(C1-C3 alkylene)-COOH;
n is an integer selected from 6 to 12.

In another preferred embodiment, the pharmaceutical composition is used for thrombosis prevention and thrombolytic therapy; or used for preventing and/or treating cerebral ischemia-reperfusion injury, inhibiting neurocyte ferroptosis caused by cerebral ischemia-reperfusion, or alleviating vascular inflammation.
In another preferred embodiment, the thrombosis is selected from the group consisting of: vascular endothelial injury induced thrombosis, arteriovenous bypass thrombosis, cerebral ischemia-reperfusion injury, or a combination thereof.
In another preferred embodiment, the cerebral ischemia-reperfusion injury comprises: cerebral infarction, cerebral edema, and/or neurocyte ferroptosis.
In another preferred embodiment, the anti-inflammatory treatment is an anti-vascular inflammation treatment.
In another preferred embodiment, the vascular inflammation includes vascular inflammation caused by high glucose and high fat.
In another preferred embodiment, the pharmaceutical composition or formulation is used for preventing and/or

treating thrombosis-related diseases.

In another preferred embodiment, the thrombosis-related disease is selected from the group consisting of: cerebral thrombosis, cerebral infarction (acute ischemic stroke) and its resulting neuronal damages in nervous tissue, cerebral edema, myocardial infarction, pulmonary embolism, or a combination thereof.

In another preferred embodiment, the thrombosis-related disease includes infarction related diseases.

In another preferred embodiment, the pharmaceutical composition or formulation is used for anti-inflammatory treatment.

In another preferred embodiment, the pharmaceutical composition or formulation is used for treating neuronal damages caused by cerebral infarction, traumatic brain injury, cerebral hemorrhage, or brain tumor surgery.

In another preferred embodiment, $R_1$ is selected from the group consisting of: hydroxyl, methoxy,

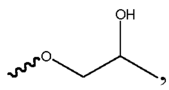, 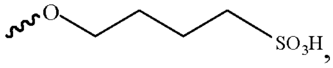, 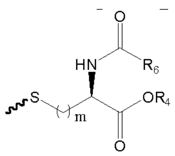 and 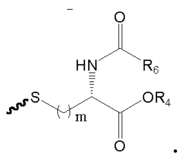.

In another preferred embodiment, m=1.

In another preferred embodiment, $R_4$ is $Na^+$.

In another preferred embodiment, $R_5$ is $CH_3$.

In another preferred embodiment, n is 6, 7, or 8.

In another preferred embodiment, $R_1$ is selected from the group consisting of: hydroxyl, methoxy,

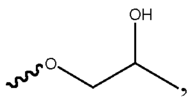, 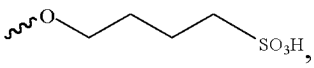, 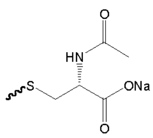 and 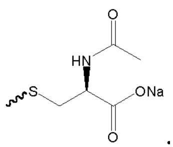.

In another preferred embodiment, $R_2$ and $R_3$ are each independently H, methyl, hydroxypropyl, hydroxyethyl or carboxymethyl.

In another preferred embodiment, the compound of formula I is selected from the group consisting of: methylcyclodextrin, carboxymethyl cyclodextrin, hydroxyethyl - β - ethylcyclodextrin, hydroxypropyl - β - cyclodextrin, and sulfobutylether-β-cyclodextrin.

In another preferred example, the parent nucleus of the compound of formula I is cyclodextrin.

In another preferred embodiment, the compound of formula I is

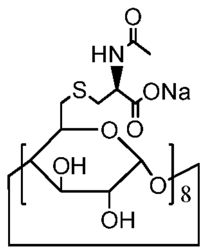

In another preferred embodiment, the compound of formula I is selected from the group consisting of:

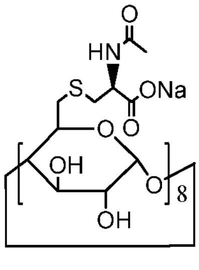 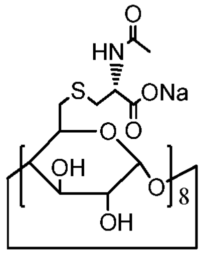

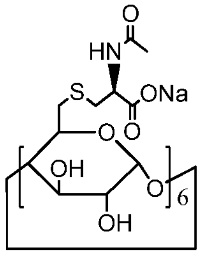
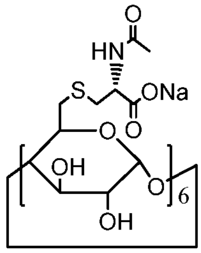

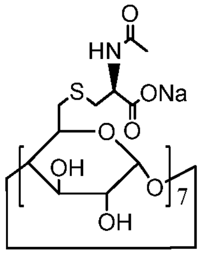
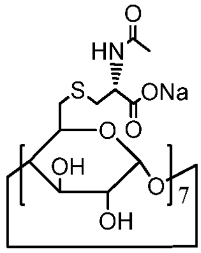

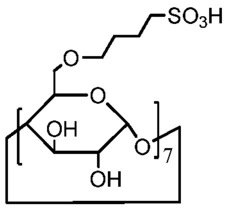
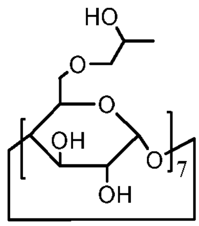

In another preferred embodiment, the pharmaceutical composition further comprises an additional active ingredient.

In another preferred embodiment, the additional active ingredient is selected from the group consisting of: butylphthalide, urokinase, edaravone, or a combination thereof.

In a preferred embodiment, the pharmaceutical composition or formulation comprises the compound of formula (I), or a pharmaceutically acceptable salt thereof as active ingredients; and pharmaceutically acceptable carriers.

In another preferred embodiment, the dosage form of the pharmaceutical composition or formulation is selected from the group consisting of: injections, lyophilized powders for injection, sustained-release, controlled release, enteric-coated tablets or capsules, granules.

In another preferred embodiment, the pharmaceutical composition or formulation contains 0.001-99wt%, preferably 0.1-90wt%, and more preferably 1-80wt% of the compound of formula I, or a pharmaceutically acceptable salt thereof as active ingredients, calculated by the total weight of the composition.

In the second aspect of the present invention, provided is a use of a combination of the compound of formula I and urokinase for the preparation of a medication for treating thrombosis-related diseases.

In the third aspect of the present invention, provided is a use of a combination of the compound of formula I and butylphthalide for the preparation of a medication for treating neurological damages caused by cerebral infarction.

In the fourth aspect of the present invention, provided is a pharmaceutical composition or formulation comprising (a) active ingredient, the active ingredient include the compound of formula I, or a pharmaceutically acceptable salt thereof; and (b) pharmaceutically acceptable carriers, the pharmaceutical composition or formulation is used for:

(a) prevention and/or treatment of thrombosis-related diseases;
(b) anti-inflammatory treatment; and/or
(c) treatment of neuronal damages caused by cerebral infarction, traumatic brain injury, cerebral hemorrhage, or brain tumor surgery.

In another preferred embodiment, when the active ingredient contains two components, the weight ratio of the two components is 1:20 to 20: 1, preferably 1: 10 to 10: 1, and more preferably 1:5 to 5:1.

In the fifth aspect of the present invention, provided is a kit, wherein the kit comprises:

(1) a first container, and a first pharmaceutical composition in the first container, the first pharmaceutical composition comprises a first compound, or a pharmaceutically acceptable salt thereof, and pharmaceutically acceptable carriers;

(2) a $n^{th}$ container, and a $n^{th}$ pharmaceutical composition in the $n^{th}$ container, the $n^{th}$ pharmaceutical composition comprises a $n^{th}$ compound, or a pharmaceutically acceptable salt thereof, and pharmaceutically acceptable carriers; wherein, n is an integer selected from 2-8;

wherein, both the first compound and the $n^{th}$ compound are compounds of formula I or pharmaceutically acceptable salts thereof, or the first compound is a compound of formula I or a pharmaceutically acceptable salt thereof, and at least one of the $n^{th}$ compound is an additional active substance, wherein the compound of formula I is as defined in claim 1;
and/or (3) optional an instruction manual.

In the sixth aspect of the present invention, provided is a method for treating diseases, the disease is as described in the first aspect of the present invention, comprising a step of: administering the compound of formula I or a pharmaceutically acceptable salt thereof to a subject in need thereof, wherein the compound of formula I is as described in claim 1.
In another preferred embodiment, the subject is mammal.
In another preferred embodiment, the subject is human.
It should be understood that, within the scope of the present invention, each of the above technical features of the present invention and each of the technical features specifically described in the following (such as the embodiments) can be combined with each other to constitute a new or preferred technical solution. Due to space limitations, It will not be repeated herein.

## EMBODIMENTS FOR CARRYING OUT THE INVENTION

After extensive and in-depth research, the inventor unexpectedly discovered for the first time that a class of compounds with structures as shown in formula I have significant effects on the prevention and/or treatment of thrombosis-related diseases; anti-inflammatory treatment; the treatment of neuronal damages caused by cerebral infarction, traumatic brain injury, cerebral hemorrhage, or brain tumor surgery. The experiment shows that the compound of formula I has good therapeutic effects on brain nerve damage caused by stroke. The compound of formula I of the present invention can be used for the prevention of thrombosis diseases, thrombolysis, anti-inflammatory treatment, etc. The present invention is completed on this basis.

## TERMS:

The term "halogen" refers to F, Cl, Br and I.
The term "C1-C6 alkyl" refers to a linear or branched chain alkyl having 1 to 6 carbon atoms, such as methyl, ethyl, propyl, isopropyl, butyl, isobutyl, tert-butyl, neopentyl and tert-pentyl and the like.
The term "C2-C6 alkenyl" refers to a linear or branched alkenyl having 2-6 carbon atoms and containing a double bond, including, without limitation, vinyl, propenyl, butenyl, isobutenyl, pentenyl, hexenyl, and the like.
The term "C2-C6 alkynyl" refers to a linear or branched alkynyl having 2-6 carbon atoms and containing a triple bond, including, without limitation, ethynyl, propynyl, butynyl, isobutynyl, pentynyl, hexynyl, and the like.
The term "C1-C6 hydroxyalkyl" refers to a linear or branched alkyl group having 1 -6 carbon atoms and containing a hydroxyl group, including, without limitation, hydroxymethyl, hydroxyethyl, hydroxypropyl, hydroxybutyl and the like. Preferably, C1-C3 hydroxyalkyl.
The term "C3-C8 cycloalkyl" refers to a cyclic alkyl having 3-8 carbon atoms on the ring, including, without limitation, cyclopropyl, cyclobutyl, cyclopentyl, cyclohexyl, cycloheptyl, cyclooctyl, and the like.
The term "C1-C6 alkoxy" refers to a linear or branched alkoxy group having 1 -6 carbon atoms, including, without limitation, methoxy, ethoxy, propoxy, isopropoxy, butoxy and the like. Preferably C1-C4 alkoxy.
The terms "aromatic ring" and "aryl" have the same meaning, preferably is "C6-C10 aryl". The term "C6-C10 aryl" refers to an aromatic ring group having 6-10 carbon atoms without any heteroatoms on the ring, such as phenyl, naphthyl and the like.
The term "heteroaryl" refers to a heteroaromatic system containing 1 to 4 heteroatoms, wherein the heteroatoms include nitrogen, oxygen, and $S(O)_r$ (wherein, r is an integer of 0, 1, 2). For example, 4-8-membered heteroaryl refers to a heteroaromatic system containing 4-8 ring atoms, and 4-10 membered heteroaryl refers to a heteroaromatic system containing 4-10 ring atoms, including but not limited to pyrrolyl, furyl, thienyl, pyrazolyl, thiazolyl, imidazolyl, oxazolyl, isoxazolyl, pyridinyl, pyranyl, pyridazinyl, pyrimidinyl, pyrazinyl, benzimidazolyl, triazolyl, etc.
Unless specifically stated, the groups described in the present invention are "substituted or unsubstituted", the groups of the present invention can be substituted by substituents selected from the group consisting of: halogen, acyloxy, cyano, amino, nitro, carboxyl, amide, carboxymethyl, C1-C6 alkyl, C1-C6 alkoxy, C1-C6 haloalkyl, C2-C6 alkenyl, C2-C6

haloalkenyl, C2-C6 alkynyl, C2-C6 haloalkynyl, hydroxyl, C3-C6 cycloalkyl, C3-C6 halocycloalkyl, hydroxy C1-C4 alkyl, C5-C7 cycloalkenyl, phenyl, naphthyl, etc.

"  " represents the binding position of the group.

## Active ingredient

As used herein, the terms "compound of the present invention", and "active ingredient of the present invention" can be used interchangeably and refer to the compound of formula I.

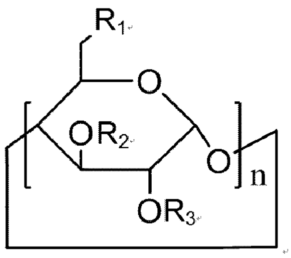

(I)

As used herein, the term also comprises pharmaceutically acceptable salt of the compound of formula I. The term "pharmaceutically acceptable salt" refers to salts that are formed of the compound of the invention and acids or bases, and suitable for use as a drug. Pharmaceutically acceptable salts include inorganic salts and organic salts. A class of preferred salts are salts formed of the polymer of the present invention and the acids. Acids suitable for forming salts include, but are not limited to, inorganic acids such as hydrochloric acid, hydrobromic acid, hydrofluoric acid, sulfuric acid, nitric acid, phosphoric acid and the like; organic acids such as methanoic acid, acetic acid, propionic acid, oxalic acid, malonic acid, succinic acid, fumaric acid, maleic acid, lactic acid, malic acid, tartaric acid, citric acid, picric acid, methane sulfonic acid, toluenesulfonic acid, benzene sulfonic acid, and the like; and acidic amino acids such as aspartic acid, glutamic acid and the like.

The compound of formula I of the present invention may be prepared by methods well known to those skilled in the art in the prior art, and there are no particular limitations on the reaction parameters of the individual steps. In addition, typical compounds of the present invention are also commercially available.

As used herein, in the compound of formula I, the chiral carbon atom, if exist, may be in the R configuration, the S configuration, or a mixture of both.

In the present invention, the active ingredient is a compound of formula I,

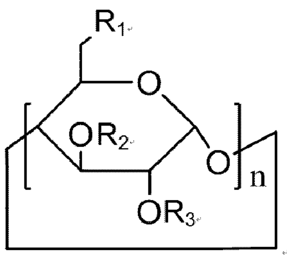

(I)

wherein,

$R_1$ is selected from the group consisting of: hydroxyl, C1-C6 alkoxy, halogen, substituted or unsubstituted -O-C1-C6 alkyl, and

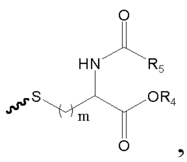
,

wherein the substituted refers to substitution of sulfonic acid group or hydroxyl;

wherein, m is an integer selected from 1 - 6;
the carbon atom of

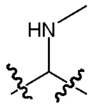 in 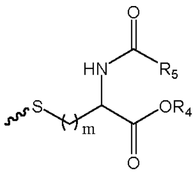

is chiral carbon atom, and the chirality is selected form the group consisting of:

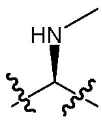 and 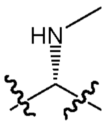;

$R_4$ is a metal ion selected from the group consisting of: $Na^+$, $K^+$, $Li^+$, and $Cs^+$;
$R_5$ is C1-C6 alkyl, C3-C8 cycloalkyl, C2-C6 alkenyl, C2-C6 alkynyl, aryl or heteroaryl;
$R_2$ and $R_3$ are each independently selected from: H, C1-C6 alkyl, C2-C6 alkenyl, C2-C6 alkynyl, C3-C8 cycloalkyl, C2-C6 hydroxyalkyl, and -(C1-C3 alkylene)-COOH;
n is an integer selected from 6 to -12.

In one embodiment, $R_1$ is selected from the group consisting of: hydroxyl, methoxy,

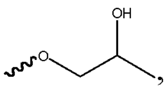, 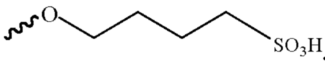, 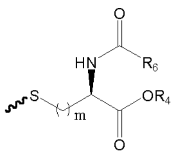 and 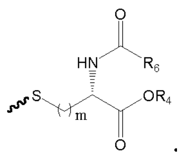.

In another embodiment, m=1.

In another embodiment, $R_4$ is $Na^+$.

In another embodiment, $R_5$ is $CH_3$.

In another embodiment, n is 6,7,or 8.

In another embodiment, $R_1$ is selected from the group consisting of: hydroxyl, methoxy,

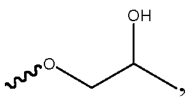, 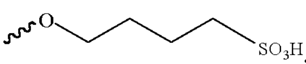, 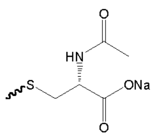 and 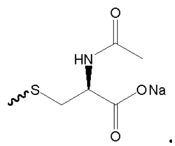.

In another embodiment, $R_2$ and $R_3$ are each independently selected from: H, methyl, hydroxypropyl, hydroxyethyl and carboxymethyl.

In another embodiment, the compound of formula I is selected from the group consisting of: methylcyclodextrin, carboxymethyl cyclodextrin, hydroxyethyl - β - ethylcyclodextrin, hydroxypropyl - β - cyclodextrin, and sulfobutylether-β-cyclodextrin.

In another example, the parent nucleus of the compound of formula I is cyclodextrin.

In another embodiment, the compound of formula I is

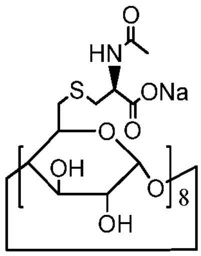

In another embodiment, the compound of formula I is selected from the group consisting of:

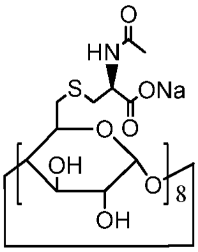

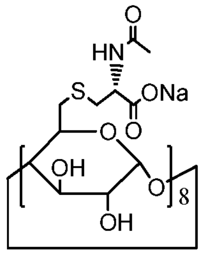

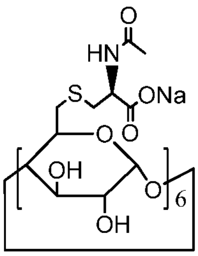

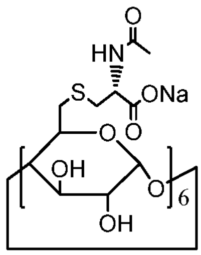

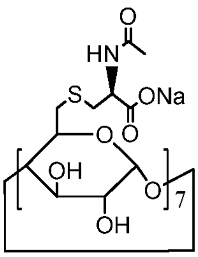

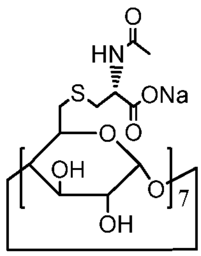

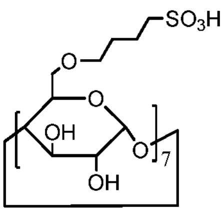

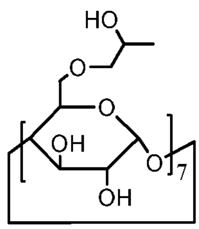

**Pharmaceutical composition and method of administration**

The present invention also provides a pharmaceutical composition comprising a pharmaceutically acceptable carrier and one or more safe and effective amounts of the compound described in the present invention.

Due to the excellent anti-thrombotic activity of the compound of the present invention, the compounds of the invention and various crystal forms, pharmaceutically acceptable inorganic or organic salts, hydrates or solvates thereof, and pharmaceutical compositions containing the the compound of the present invention as active ingredients can be used in the treatment, prevention and alleviation of the diseases associated with embolism.

The pharmaceutical composition of the present invention comprises a safe and effective amount of the compound of the present invention, or a pharmaceutically acceptable salt thereof and pharmaceutically acceptable

excipients or carriers. Wherein "safe and effective amount" refers to an amount of compound which is sufficient to significantly improve the condition, and not to generate severe side effects. Generally, the pharmaceutical composition contains 1-2000 mg polymorphs of the invention per dose, preferably, 10-1000 mg polymorphs of the invention per dose. Preferably, the "one dose" is one capsule or one pill.

"Pharmaceutically acceptable carrier" means one or more compatible solid or liquid fillers or gelatinous materials which are suitable for human use and should be of sufficient purity and sufficiently low toxicity. "Compatible" used herein refers to the ability of each component of a composition can be mixed with the compound of the present invention and can be mixed with each other without appreciably reducing the efficacy of the compound. Examples of pharmaceutically acceptable carrier include cellulose and derivatives thereof (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween®), wetting agent (such as lauryl sodium sulfate), colorant, flavoring, stabilizer, antioxidant, preservative, pyrogen-free water, etc.

The pharmaceutical composition is injections, capsules, tablets, pills, powders or granules.

There is no special limitation of administration mode for the compound or pharmaceutical compositions of the present invention, and the representative administration mode includes (but is not limited to): oral, intratumoral, rectal, parenteral (intravenous, intramuscular or subcutaneous), and topical administration.

The solid dosage forms used for oral administration include capsules, tablets, pills, powders, and granules. In these solid dosage forms, the active compounds are mixed with at least one conventional inert excipient (or carrier), such as sodium citrate or CaHPO4, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, calciumcetyl alcohol, magnesium stearate, solid polyethylene glycol, sodium lauryl sulfate, or the mixtures thereof. In capsules, tablets, and pills, the dosage form may also include buffers.

Solid dosage forms such as tablets, sugar pills, capsules, pills, and granules can be prepared using coating and shell materials, such as casings and other materials well-known in the art. They can contain opacifiers, and the release of active compounds or compounds in the composition can be delayed in a certain part of the digestive tract. Examples of embedding components that may be employed are polymeric substances and waxes. If necessary, the active compound may also be formed into a microcapsules with one or more of the above excipients.

Liquid dosage forms for oral administration include pharmaceutically acceptable lotion, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

In addition to these inert diluents, the composition can also include additives such as wetting agents, emulsifiers and suspensions, sweeteners, correctors, and spices.

In addition to active compounds, suspensions can include suspending agents such as ethoxylated isooctadecanol, polyoxyethylene sorbitol and dehydrated sorbitol esters, microcrystalline cellulose, methanol aluminum and agar, or mixtures of these substances.

Compositions for parenteral injection may include physiologically acceptable sterile aqueous or anhydrous solutions, dispersion liquid, suspensions or lotions, and sterile powders for re dissolution into sterile injectable solutions or dispersions. Suitable aqueous and non-aqueous carriers, diluents, solvents or excipients include water, ethanol, polyols, and their suitable mixtures.

The dosage forms of the compounds of the present invention used for local administration include ointments, powders, patches, sprays, and inhalants. The active ingredients are mixed under sterile conditions with physiologically acceptable carriers and any preservatives, buffers, and propellants if necessary.

The compound of the present invention may be administered alone or in combination with other pharmaceutically acceptable compound (such as anti-thrombotic drugs).

The treatment method of the present invention can be administered alone or in combination with other treatment methods or therapeutic drugs.

When the pharmaceutical compositions are used, a safe and effective amount of compound of the present invention is applied to a mammal (such as human) in need of, wherein the dose of administration is a pharmaceutically effective dose. For a person weighed 60 kg, the daily dose is usually 1- 2000 mg, preferably 50-1000 mg. Of course, the particular dose should also depend on various factors, such as the route of administration, patient healthy status, which are within the skills of an experienced physician.

**The main advantages of the present invention include:**

(a) The compound of formula I of the present invention has excellent therapeutic effects on brain nerve damages caused by stroke.
(b) The compound of formula I of the present invention can be used for the prevention of thrombosis diseases, and the treatment of thrombolysis, without causing bleeding.
(c) The compound of formula I of the present invention has anti-inflammatory effect.
(d) Compound 1 in the compound of formula I of the present invention has excellent safety performance and low toxic side effects.

The present invention was further described hereafter in combination with specific embodiments. It should be understood that these examples are only used to illustrate the and not to limit the scope of the invention. The experimental methods with no specific conditions described in the following examples are generally performed under the conventional conditions, for example, according to J. Sambrook et al., Molecular Cloning: A Laboratory Manual, 3rd Edition, Science Press, 1989, or according to the manufacture's instructions. Unless otherwise stated, percentages and parts are percentages by weight and parts by weight.

**Example 1: Evaluation experiment of the preventative effect of sample on vascular endothelial injury induced thrombosis**

**Experimental method:** 420 tails of 5 dpf wild type AB strain zebrafish were randomly selected and placed in a six-well plate, with 30 fish per well (experimental group). The fish were injected with compound (1), compound (2), compound (3), compound (4), compound (5), compound (6), compound (7), compound (8), compound (9), compound (10), and compound (11) (structural formula shown in Table 1) at a dose of 4.00 ng/tail, with positive control drug acetylsalicylic acid being administered by dissolving it in water at a concentration of 30.0 μ g/mL. Normal control group (i.e. zebrafish treated with standard diluted water) and model control group were set up, with a capacity of 3 mL per well (experimental group). Except for normal control group, the remaining experimental groups were all given Punatinib by dissolving it in water to induce vascular endothelial injury induced thrombosis model in zebrafish. The zebrafish were stained with o-dianisidine after co-treatment with ponatinib and samples for a period of time under incubation conditions at 28 °C.

After staining, 10 zebrafish in each group were randomly selected and photographed under the microscope, and the intensity of cardiac erythrocyte staining in zebrafish was analyzed using NIS-Elements D 3.20 advanced image-processing software, and the samples were evaluated for the preventative effect on vascular endothelial injury induced thrombosis by the statistical results of cardiac erythrocyte staining intensity.

The preventative effect on thrombosis is calculated as follows:

$$\text{Preventative effect on thrombosis (\%)}=\frac{[\text{S(tset product group)}-\text{S(model control group)}]}{[\text{S(normal control group)}-\text{S(model control group)}]}\times 100\%$$

Statistical analysis was performed using SPSS software, and $p < 0.05$ indicates significant differences

**Experimental results:**

**Table 1: Preventative effect of samples on vascular endothelial injury induced thrombosis (n=10)**

| Group | Sample structural formula/name | Dose (ng/tail) | Cardiac erythrocyte staining intensity (pixels, mean ± SE) | Preventative effect on thrombosis (%) |
|---|---|---|---|---|
| **Normal control group** | -- | - | 5312 ± 318*** | - |
| **Model control group** | -- | - | 2597 ± 251 | - |
| **Acetylsalicylic acid** | -- | 30.0 μg/mL | 5345 ± 228*** | 101 |

(continued)

| Group | Sample structural formula/name | Dose (ng/tail) | Cardiac erythrocyte staining intensity (pixels, mean ± SE) | Preventative effect on thrombosis (%) |
|---|---|---|---|---|
| **Compound (1)** | 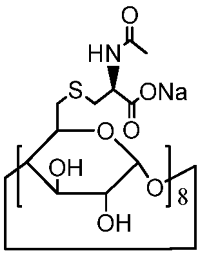 CAS: 1309580-41-3 | | 4397 ± 454** | 66 |
| **Compound (2)** | 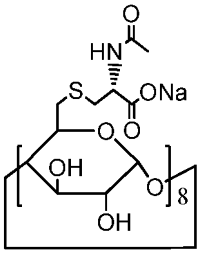 | | 3655 ± 318* | 39 |
| **Compound (3)** | 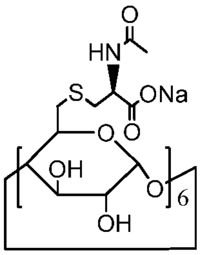 | | 3045 ± 219 | 17 |
| **Compound (4)** | 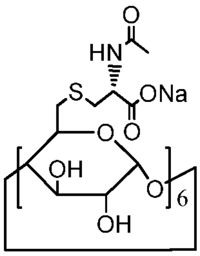 | 4.00 ng/tail | 4300 ± 195*** | 63 |
| **Compound (5)** | 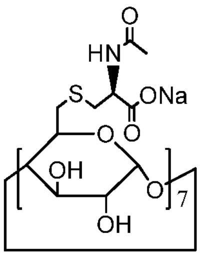 | | 4340 ± 255*** | 64 |

(continued)

| Group | Sample structural formula/name | Dose (ng/tail) | Cardiac erythrocyte staining intensity (pixels, mean ± SE) | Preventative effect on thrombosis (%) |
|---|---|---|---|---|
| **Compound (6)** | 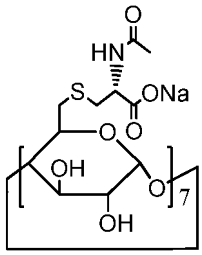 | | 4938 ± 436*** | 86 |
| **Compound (7)** | 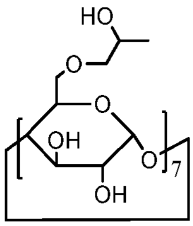 | | 4584 ± 271*** | 73 |
| **Compound (8)** | 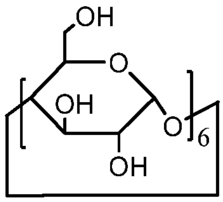 | | 4580 ± 291*** | 73 |
| **Compound (9)** | 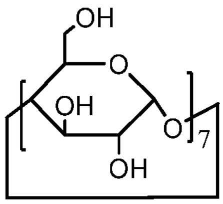 | | 4709 ± 296*** | 78 |
| **Compound (10)** | 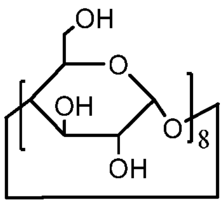 | | 4326 ± 383** | 64 |
| **Compound (11)** | 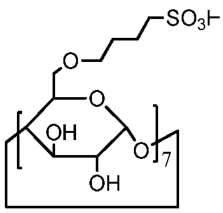 | | 4749 ± 124*** | 79 |

compared with model control group, *p<0.05, ** p<0.01, *** p ≤ 0.001.

**Experimental results:** the intensity of cardiac erythrocyte staining of zebrafish in the 4.00 ng/tail dose group was 4397, 3655, 3045, 4300, 4340, 4938, 4584, 4580, 4709, 4326 and 4749 pixels respectively for compound (1), compound (2), compound (3), compound (4), compound (5), compound (6), compound (7), compound (8), compound (9), compound (10), and compound (11).

Compared to model control group, the preventative effect on thrombosis of compound (3) in 4.00 ng/tail dose

group was 17% , with $p > 0.05$. The preventative effect on thrombosis of compound (1), compound (2), compound (4), compound (5), compound (6), compound (7), compound (8), compound (9), compound (10) and compound (11) in 4.00 ng/tail dose group were 66%, 39%, 63%, 64%, 86%, 73%, 73%, 78%, 64%, and 79%, respectively, with $p < 0.01$, $p < 0.05$, $p < 0.001$, $p < 0.001$, $p < 0.001$, $p < 0.001$, $p < 0.001$, $p < 0.001$, $p < 0.01$, $p < 0.001$, respectively.

**Conclusion:** Compound (1), Compound (2), Compound (4), Compound (5), Compound (6), Compound (7), Compound (8), Compound (9), Compound (10), and Compound (11) showed significant prophylactic effects against endothelial injury induced thrombosis caused by ponatinib in zebrafish under the conditions of this experiment.

Especially, in 4.00 ng/tail dose group, compounds (6), (11), (9), (7), (8), and (1) had a very significant preventative effect against vascular endothelial injury induced thrombosis caused by ponatinib in zebrafish, with thromboprophylaxis (%) index ranging from 66 to 86%, while the thromboprophylaxis (%) index of 30ug/ml of acetylsalicylic acid was 101%.

**Example 2** Evaluation on sensitization risk induced by the sample

**Experimental method:** 2 dpf wild type AB strain zebrafish were randomly selected and placed in a 24-well plate, with each group 6 parallel wells and 10 zebrafish per well. The zebrafish were injected intravenously with samples at a dose of 500 ng/tail and the positive control "C48/80" at a concentration of 1.5 μg/mL, while normal control group was set up. Each well has a capacity of 1 mL.

All experimental groups were given BAPNA by dissolving it in the water. After treatment at 28°C for 1 day, the liquid was transferred to 96-well plates with 200 μL/well, and the relative absorbance values (OD values) of the expression levels of tryptase in each experimental group were detected using an microplate reader, and the results of the statistical analysis of the OD values were used to evaluate the risk of sensitization by sample. Statistical results were expressed as mean ± SE.

$$\text{sensitization risk}(\%) = \frac{[\text{OD}(\text{sample group}) - \text{OD}(\text{normal control group})]}{\text{OD}(\text{normal control group})} \times 100\%$$

Statistical analysis was performed using SPSS software, and $p < 0.05$ indicates significant differences.

**Table 2 Sensitization risk of each sample**

| Evaluation index | | Normal control group | Positive control group | Compound (1) | Compound (2) | Compound (10) |
|---|---|---|---|---|---|---|
| Absorbance value (OD value) | 1 | 0.05085 | 0.11145 | 0.06575 | 0.04785 | 0.07585 |
| | 2 | 0.05905 | 0.09885 | 0.05845 | 0.05085 | 0.06795 |
| | 3 | 0.05395 | 0.09815 | 0.05605 | 0.05405 | 0.06445 |
| | 4 | 0.05515 | 0.09295 | 0.05725 | 0.05655 | 0.07115 |
| | 5 | 0.05305 | 0.09545 | 0.05895 | 0.05255 | 0.06947 |
| | 6 | 0.05385 | 0.09985 | 0.06195 | 0.04865 | 0.06795 |
| | mean | 0.054 | 0.099 | 0.060 | 0.052 | 0.069 |
| | SE | 0.001 | 0.003 | 0.001 | 0.001 | 0.002 |
| | Sensitization risk (%) | | 83.3 | 11.1 | -3.7 | 27.8 |
| | Relative sensitization risk (compared with positive control group) | | 100% | 13% | -4% | 33% |
| | P | | < 0.001 | < 0.05 | > 0.05 | < 0.001 |

**Experimental results:** The sensitization risk of the compounds of the present invention tested was low, even under the condition that the dose was increased by 125 times (i.e. 500 ng/tail) based on the dose of 4ng/tail in Example 1. Representatively, as shown in Table 2, the sensitization risk of the tested compound (1), compound (2), and compound (10) of the present invention were all significantly lower than the that of the positive control.

Among them, "Compound (2)" and "Compound (1)" have no or substantially no sensitization risk, and "Compound (10)" has a low sensitization risk.

**Experimental conclusion:** Combining the experimental results of Examples 1 and 2, i.e., considering the safety and efficacy in a comprehensive manner, compound (1) was selected as the preferred compound for subsequent experiments.

**Example 3 Evaluation of the preventative effect of compound 1 on vascular endothelial injury induced thrombosis**

**Experimental method:** Wild-type AB strain zebrafish were treated with 4 μg/mL punatinib for 18 h to establish vascular endothelial-injury thrombosis model in zebrafish. 150 of 5 dpf wild-type AB strain zebrafish were randomly selected and placed in six-well plates with 30 fish per well (experimental group). The zebrafish were injected with compound (1) at a dose of 2.06ng/tail, and 6.18 ng/tail, and the positive control drug acetylsalicylic acid was given by dissolving it in water with a concentration of 30 μg/mL, while the normal control group (i.e., zebrafish treated with standard dilution water) and the model control group were set up, and the volume of each well (experimental group) was 3 mL. Except for normal control group, the remaining experimental groups were all given Punatinib by dissolving it in water to induce vascular endothelial injury thrombosis model in zebrafish. The zebrafish were co-treated with compound (1) and punatinib for a period of time under incubation condition at 28°C, and then stained with o-dianisidine. After staining, 10 zebrafish were randomly selected from each group and photographed under the microscope. The intensity of cardiac erythrocyte staining in zebrafish was analyzed using NIS-Elements D 3.10 advanced image-processing software, and the preventative effect of compound (I) on vascular endothelial injury thrombosis was evaluated based on the statistical results of cardiac erythrocyte staining intensity. The preventative effect on thrombosis is calculated as follows:

$$\text{Thromboprophylactic effect (\%)}=\frac{[\text{S(tset product group)}-\text{S(model control group)}]}{[\text{S(normal control group)}-\text{S(model control group)}]}\times 100\%$$

Statistical analysis were performed using Analysis of Variance and Dunnett's t-test, and $p < 0.05$ indicates a significant difference, providing a representative experimental plot.

**Table 3: Preventative effect of compound (I) on vascular endothelial injury induced thrombosis (n=10)**

| Group | Dose (ng/tail) | Cardiac erythrocyte staining intensity (pixels, mean ± SE) | Thromboprophylactic effect (%) |
|---|---|---|---|
| Normal control group | - | 5080 ± 155*** | 100 |
| Model control group | - | 2300 ± 237 | 0 |
| Acetylsalicylic acid | 30 μg/mL | 4655 ± 136*** | 85 |
| Compound (1) | 2.06 | 4601 ± 265*** | 83 |
| | 6.18 | 5044 ± 345*** | 99 |

Compared with model control group, *** p<0.001.

**Experimental results:** The intensity of cardiac erythrocyte staining in zebrafish in compound (1) 2.06 ng/tail and 6.18 ng/tail dose groups were 4601 and 5044 pixels, respectively, with $p < 0.001$ compared with the model control group, and the thromboprophylactic effect thereof were 83% and 99%, respectively.

**Experimental Conclusion:** Compound (1) had a significant and dose-dependent preventative effect on punatinib-induced vascular endothelial injury thrombosis in zebrafish, and the preventative effect of compound (1) on vascular endothelial injury thrombosis was superior to that of the positive control, acetylsalicylic acid.

**Example 4 Effect of Compound (1) on Arteriovenous Bypass Thrombosis in Rats**

**Experimental method:**

Grouping: Divided into 5 groups, with 10 animals in each group. The model control group was given with solvent, and the test drug compound (1) and the positive control were given intravenously once a day for consecutive 3 d. The modeling started 30 min after the last dose.

Model preparation: 50 SD male rats were anesthetized with sodium pentobarbital at a dose of 60 mg/kg via ip. The rat was fixed in the supine position, and the skin was incised at the midline of the neck; the fascia and muscle tissues were separated, the left common carotid artery and the right external jugular vein were dissociated, and the arterio-venous (A-V) bypass surgery was performed using the polyethylene tubing with a built-in thread. The arterial clip was used to clamp the proximal end of the carotid artery, and one end of the polyethylene tubing was inserted into the artery, and another end was inserted into the vein, thereby completing the bypass surgery. 30 minutes after the last dose, the arterial clip was opened, and blood flowed from the left common carotid artery to the right external jugular vein through the bypass tubing, and the blood flow formed a bypass circulation, and the thrombus was taken out 15 min later.

**Detection index:** the blood flow was open after 15 min, the thread was taken out, the residual liquid was sucked dry, the thread attached with thrombus was weighed and the wet weight of thrombus was calculated; the dry weight of thrombus was weighed and calculated after bake-drying in the oven at 50 °C. Thrombosis inhibition rate was calculated. Tails were clipped at the end of the experiment to detect bleeding time.

**Table 4 Effect of compound (1) on arteriovenous bypass thrombosis in rats ($\bar{x}\pm s$, n=10)**

| Group | Dose mg/kg | Wet weight of thrombus | Dry weight of thrombus | Wet weight inhibition rate (%) | Dry weight inhibition rate (%) |
|---|---|---|---|---|---|
| Model control | - | 0.0319 ± 0.0033 | 0.0082 ± 0.0017 | | |
| Compound (1) | 10 | 0.0254 ± 0.0042** | 0.0069 ± 0.0014 | 19.4 | 15.9 |
| Compound (1) | 20 | 0.0246 ± 0.0045** | 0.006 ± 0.0014** | 21.9 | 26.8 |
| Compound (1) | 40 | 0.0207 ± 0.0041*** | 0.0051 ± 0.0011*** | 34.3 | 37.8 |
| Ozagre | 16 | 0.0208 ± 0.0041*** | 0.0054 ± 0.0018** | 34 | 34.1 |
| Note: Compared with model group, *P<0.05, **P<0.01, ***P<0.001; | | | | | |

**Results:** Compared with the model control group, intravenous injection of compound (1) at a dose of 10 mg/kg, 20 mg/kg, 40 mg/kg inhibit rat arteriovenous bypass thrombosis in different degrees, and the thrombus wet weight and dry weight inhibition rate (%) were respectively 19.4% (P>0.005) and 15.9% (P>0.005); 21.9% (P<0.001) and 26.8%(P<0.001); 34.3% (P<0.001) and 37.8% (P<0.001). The thrombus wet weight and dry weight inhibition rate (%) of Ozagre administered intravenously at a dose of 16 mg/kg were 34%(P<0.001) and 34.1%(P<0.01).

**Conclusion:** The anti-thrombotic effect of Compound (1) is dose-dependent, and is comparable or superior to that of ozagrel.

**Table 5 Effect of compound (1) on bleeding time in rats ($\bar{x}\pm$ s, n=10)**

| Group | Dose (mg/kg) | Bleeding time (s) |
|---|---|---|
| Model control | - | 396.1 ± 143.9 |
| Compound (1) | 10 | 403.4 ± 185.1 |
| Compound (1) | 20 | 422.6 ± 205.7 |
| Compound (1) | 40 | 428.4 ± 185.6 |
| Ozagre | 16 | 740.8 ± 224*** |
| Note: Compared with model group, ***P<0.001. | | |

**Experimental results:** Compared with the model control group, intravenous administration of different doses of compound (1) have no significant effect on the bleeding time in rats; positove control, intravenous administration of Ozagre, significantly prolonged the bleeding time in rats (P<0.01).

**Experimental conclusion:** The bleeding experiment of compound (1) shows non dose dependence, which

proves that compound (1) has no significant bleeding risk.

**Example 5 Effect of preventative administration of compound (1) on cerebral ischemia-reperfusion injury in rats**

**Experimental method:** SD rats were randomly divided into six groups, i.e., pseudosurgery group, model control group, positive control drug ozagre (6 mg/kg) group, and administration drug compound (1) high-dose (67.5 mg/kg) group, medium-dose (22.5 mg/kg) group, and low-dose (7.5 mg/kg) group. The administration group was pre-administered via tail vein injection, while the pseudosurgery group and model control group were given equal volumes of physiological saline once a day for 3 consecutive days. 10 minutes after the last administration, a rat MCAO/R model was established and performed reperfusion 2 hours post ischemia. 24 hours after MCAO reperfusion, the neurobehavioral scores of rats were performed to determine their neurological function; the cerebral infarction rate and cerebral water content in rats were determined using 2,3,5-triphenyltetrazolium chloride (TTC) staining method.

**Table 6 Effect of preventative administration of compound (1) on cerebral infarction rate in rats with focal cerebral ischemia (MCAO)/reperfusion (The data were expressed in mean ± SD, n=8)**

| Group | Dose (mg/kg) | Cerebral infarction rate (%) |
|---|---|---|
| Pseudo surgery group | - | 0 |
| Model control group | - | 27.59 ± 6.52 |
| Ozagre group | 6mg/kg | 14.39 ± 8.14** |
| Compound (1) high-dose group | 67.5mg/kg | 12.28 ± 10.29** |
| Compound (1) medium-dose group | 22.5mg/kg | 13.93 ± 6.99** |
| Compound (1) low-dose group | 7.5mg/kg | 15.18 ± 4.74* |
| **P<0.01, compared with model control group; *P<0.05, compared with model control group. | | |

**Table 7 Effect of preventative administration of compound (1) on cerebral water content in rats with focal cerebral ischemia (MCAO)/reperfusion**

| (The data were expressed in mean ± SD, n=8) | | |
|---|---|---|
| Group | Dose (mg/kg) | Cerebral water content (%) |
| Pseudosurgery group | - | 77.18 ± 0.51 |
| Model control group | - | 82.12±1.28## |
| Ozagre group | 6mg/kg | 78.42 ± 3.73 * * |
| Compound (1) high-dose group | 67.5mg/kg | 78.23 ± 1.84** |
| Compound (1) medium-dose group | 22.5mg/kg | 79.01 ± 1.27* |
| Compound (1) low-dose group | 7.5mg/kg | 80.16 ± 1.03 |
| ##P<0.01, compared with pseudosurgery group; **P<0.01, compared with model control group; *P<0.05, compared with model control group. | | |

**Table 8 Effect of preventative administration of compound (1) on neuroethology of rats with focal cerebral ischemia (MCAO)/reperfusion**

| (The data are expressed in mean ± SD, n=8) | | |
|---|---|---|
| Group | Dose (mg/kg) | Behavioral score |
| Pseudosurgery group | - | 0 |
| Model control group | - | 3.00 ± 0.76 |
| Ozagre group | 6mg/kg | 1.50 ± 0.56** |
| Compound (1) high-dose group | 67.5mg/kg | 1.50 ± 0.53** |
| Compound (1) medium-dose group | 22.5mg/kg | 1.87 ± 0.64* |

(continued)

(The data are expressed in mean ± SD, n=8)

| Group | Dose (mg/kg) | Behavioral score |
|---|---|---|
| Compound (1) low-dose group | 7.5mg/kg | 2.00 ± 1.07 |
| **P<0.01, compared with model control group; *P<0.05, compared with model control group. | | |

**Results:** Compound (1) in all dose groups and the positive drug ozagre group reduced neurological function scores, cerebral infarction rate and cerebral water content of MCAO/R rats .

**Conclusion:** preventative administration of compound (1) reduces cerebral infarction rate and cerebral water content of cerebral ischemia-reperfusion injury in rats, and improves neuromotor function in rat.

**Example 6 Effect of combination of compound (1) and urokinase (i.v) on ischemic brain injury in rats caused by autologous thrombus and thrombin**

**Experimental method:** A model of cerebral ischemic injury in SD rats was prepared by injecting autologous thrombus and thrombin into the internal carotid via the external carotid artery to embolize the cerebral artery of rats, and the rats were randomly divided into five groups according to behavioral scores: the sham operated group (isolating the external carotid artery only); the model control group (the two groups were administered with equal volume of physiological saline); the urokinase (5,000 U/kg) group, and the compound (1) (22.5mg/kg) group, with 20 rats for each group (10 for the detection of cerebral infarction rate and 10 for pathological test). The drug was administered once by slow tail vein injection (1 ml per minute) 2 h after modeling, and the decrease in blood flow was monitored after the injection of thrombin, and 120 min after the administration of the drug. Neurobehavioral scoring was performed to determine the neurological function of rats after 24 h. The cerebral infarction rate and cerebral water content of rats were determined using 2,3,5-triphenyltetrazolium chloride (TTC) staining method, and the pathological damage of the brain of rats administered with drug 2 h after cerebral ischemia was observed by hematoxylin-eosin (HE) staining.

**Experimental results:**

**Table 9 Effect of compound (1) (i.v) on cerebral blood flow after cerebral ischemic injury in rats caused by autologous thrombus and thrombin**

(The data were expressed in mean ± SD, n=10)

| Group | Dosage | Percentage decrease after injection of thrombin (%) | Percentage decrease two hours after administration (%) |
|---|---|---|---|
| Blank group | - | 0.04 ± 4.01 | 0.33 ± 0.33 |
| Model control group | - | 44.40 ± 5.22 | 41.18 ± 5.67 |
| Compound (1) group | 22.5mg/kg | 43.67 ± 7.17 | 27.14±6.38**## |
| Urokinase group | 5000U/kg | 50.10 ± 7.94 | 37.12±4.49## |
| Compound (1) + Urokinase group | 22.5mg/kg+5000U/kg | 33.79 ± 3.95 | 20.14±3.77**## |
| **P<0.01, compared with model control group; ##P<0.01, compared with self group after thrombin injection. | | | |

**Table 10 Effect of compound (1) (i.v) on neurobehavior of rats (The data are expressed in mean ± SD, n=10)**

| Group | Dosage | Behavioral score |
|---|---|---|
| Model control group | - | 3.00 ± 0.82 |
| Compound (1) group | 22.5mg/kg | 1.60 ± 0.70* |
| Urokinase group | 5000U/kg | 1.60 ± 0.70* |

(continued)

| Group | Dosage | Behavioral score |
|---|---|---|
| Compound (1) + Urokinase group | 22.5mg/kg+5000U/kg | 1.60 ± 0.52* |
| *P<0.05, compared with model control group. | | |

**Table 11 Effect of compound (1) (i.v) on cerebral infarction rate and cerebral water content of rate with cerebral ischemic injury caused by autologous thrombus and thrombin**

| Group | Dosage | Cerebral infarction rate (%) | Cerebral water content (%) |
|---|---|---|---|
| Blank group | - | -- | 79.32 ± 0.63 |
| Model control group | - | 29.94 ± 6.18 | 82.20±1.33## |
| Compound (1) group | 22.5mg/kg | 13.91 ± 10.78* | 79.93 ± 1.98** |
| Urokinase group | 5000U/kg | 9.72 ± 9.89** | 79.78 ± 0.72** |
| Compound (1) + Urokinase group | 22.5mg/kg+5000U/kg | 6.61 ± 6.14** | 79.51 ± 0.81** |
| ##P<0.01, compared with blank group; *P<0.05, **P<0.01, compared with model control group. | | | |

**Table 12 Effect of compound (1) (i.v) on the pathological damage score of cerebral ischemic area in rats with cerebral ischemic injury induced by autologous thrombus and thrombin (mean±SD, n=10)**

| Group | Dosage | Pathological damage score |
|---|---|---|
| Model control group | - | 3.70 ± 0.48 |
| Compound (1) group | 22.5mg/kg | 1.90 ± 1.10* |
| Urokinase group | 5000U/kg | 1.75 ± 0.86** |
| Compound (1) + Urokinase group | 22.5mg/kg+5000U/kg | 1.55 ± 0.60** |
| *P<0.05, **P<0.01, compared with model control group. | | |

Intravenous administration of compound (1), urokinase and compound (1) + urokinase significantly reduced the percentage decrease in cerebral blood flow in the model rats, the area of cerebral infarction and cerebral water content of the rats, and improved the pathological injury and behavioral changes after the model of cerebral ischemic injury caused by autologous thrombus and thrombin, with the compound (1) + urokinase group showing the most significant level of improvement in cerebral ischemic injury caused by autologous thrombus and thrombin.

**Conclusion:** compound (1)(i.v) and its co-administration with urokinase reduce the cerebral infarction rate, cerebral water content and pathologic damage in rats with ischemic brain injury induced by autologous thrombus and thrombin, and improved the neuromotor function of rats, which shows good anti-ischemic brain injury effect.

**Example 7 Effect of therapeutic administration of compound (1) on cerebral ischemia-reperfusion injury in rats**

**Experimental method:** SD rats were randomly divided into 11 groups, including pseudosurgery group, a model control group, positive control drug edaravone via intravenous injection (6 mg/kg, i.v.) group, positive control drug butylphthalide injection via intravenous injection (5 mg/kg, i.v.) group, compound (1) high-dose (24 mg/kg, i.v.) , medium-dose (12 mg/kg, i.v.), low-dose (6 mg/kg, i.v.) via intravenous injection group, positive drug butylphthalide soft capsule via intragastric administration (60mg/kg, i.g.) group, and compound via intragastric administration at (1) high-dose (60 mg/kg, i.g.), medium-dose (30 mg/kg, i.g.), low-dose (15 mg/kg, i.g.) group. A middle cerebral artery occlusion (MCAO) model for cerebral ischemia-reperfusion of male rats was prepared using the internal carotid suture-occluded method with an ischemic time of 90 min followed by reperfusion. Injection and oral administration for once were performed 1h after reperfusion, and neurobehavioral scoring was performed on rats after 24 h to determine neurological function in rats; the cerebral infarction rate and cerebral water content of rats were determined by 2,3,5-triphenyltetrazolium chloride (TTC) staining.

**Example 13 Effect of therapeutic administration of compound (1) on cerebral infarction rate in rats with focal cerebral ischemia (MCAO)/reperfusion**

**(The data are expressed in mean ± SD, n=8)**

| Group | Dose (mg/kg) | Cerebral infarction rate (%) |
|---|---|---|
| Pseudo surgery group | - | - |
| Model control group | - | 41.33 ± 2.67 |
| Edaravone group | 6mg/kg | 10.23 ± 7.87** |
| Butylphthalide injection group | 5mg/kg | 19.18 ± 12.94* |
| Compound (1) i.v. high-dose group | 24mg/kg | 11.64 ± 9.38** |
| Compound (1) i.v. medium-dose group | 12mg/kg | 16.94 ± 9.52** |
| Compound (1) i.v. low-dose group | 6mg/kg | 30.03 ± 7.79 |
| Butylphthalide soft capsule group i.g | 60mg/kg | 15.13 ± 11.43** |
| Compound (1) i.g. high-dose group | 60mg/kg | 13.61 ± 10.24** |
| Compound (1) i.g. medium-dose group | 30mg/kg | 21.52 ± 11.04* |
| Compound (1) i.g. low-dose group | 15mg/kg | 29.49 ± 5.93 |

**P<0.01, compared with model control group; *P<0.05, compared with model control group.

**Table 14 Effect of therapeutic administration of compound (1) on cerebral water content in rats with focal cerebral ischemia (MCAO)/reperfusion**

**(The data are expressed in mean ± SD, n=8)**

| Group | Dose (mg/kg) | Cerebral water content (%) |
|---|---|---|
| Pseudo surgery group | - | 78.55 ± 0.40 |
| Model control group | - | 84.45±0.54## |
| Edaravone group | 6 mg/kg | 79.81 ± 1.03** |
| Butylphthalide injection group | 5 mg/kg | 80.56 ± 2.07* |
| Compound (1) i.v. high-dose group | 24 mg/kg | 79.95 ± 1.16** |
| Compound (1) i.v. medium-dose group | 12 mg/kg | 80.74 ± 0.89** |
| Compound (1) i.v. low-dose group | 6mg/kg | 82.30 ± 1.04* |
| Butylphthalide soft capsule group i.g | 60mg/kg | 80.10 ± 1.49** |
| Compound (1) i.g. high-dose group | 60mg/kg | 79.87 ± 1.71** |
| Compound (1) i.g. medium-dose group | 30mg/kg | 80.61 ± 1.21* |
| Compound (1) i.g. low-dose group | 15mg/kg | 82.24 ± 1.49 |

##P<0.01, compared with pseudosurgery group; **P<0.01, compared with model control group; *P<0.05, compared with model control group.

**Table 15 Effect of preventative administration of compound (1) on neuroethology in rats with focal cerebral ischemia (MCAO)/reperfusion**

**(The data were expressed in mean ± SD, n=8)**

| Group | Dose (mg/kg) | Behavioral score |
|---|---|---|
| Pseudosurgery group | - | - |
| Model control group | - | 3.00 ± 0.76 |

(continued)

**(The data were expressed in mean ± SD, n=8)**

| Group | Dose (mg/kg) | Behavioral score |
|---|---|---|
| Edaravone group | 6mg/kg | 1.62 ± 0.51** |
| Butylphthalide injection group | 5mg/kg | 1.62 ± 0.52* |
| Compound (1) i.v. high-dose group | 24mg/kg | 1.62 ± 0.52** |
| Compound (1) i.v. medium-dose group | 12mg/kg | 1.88 ± 0.35** |
| Compound (1) i.v. low-dose group | 6mg/kg | 2.13 ± 0.35 |
| Butylphthalide soft capsule group i.g | 60mg/kg | 1.50 ± 0.53** |
| Compound (1) i.g. high-dose group | 60mg/kg | 1.62 ± 0.52** |
| Compound (1) i.g. medium-dose group | 30mg/kg | 2.00 ± 0.53* |
| Compound (1) i.g. low-dose group | 15mg/kg | 2.25 ± 0.71 |

**P<0.01, compared with model control group; *P<0.05, compared with model control group.

**Results:** All dose groups of compound (1) and all positive drug groups reduced the neurological function scores, cerebral infarction rate and cerebral water content of MCAO/R rats to different degrees, with compound (1) (24 mg/kg, i.v.) and compound (1) (60 mg/kg, i.g.) groups showing the most significant effect.

**Conclusion:** therapeutic administration of compound (1) reduces the cerebral infarction rate, cerebral water content in rats with cerebral ischemia/reperfusion injury, , and improves neuromotor function in rats.

### Example 8 Study on inhibition of compound 1, compound 3 and compound 5 against neurocyte ferroptosis during cerebral ischemia/reperfusion

**Experimental method:** Primary neuron cells were incubated in a medium containing Erastin (final concentration of 50 μ M) for 24 hours, and then incubated in a medium containing different concentrations of the test drug and Erastin (final concentration of 50 μ M) for another 24 hours. After incubation, Real time PCR was used to detect mRNA expression of GPX4 in each group of cells, CCK8 assay was used to detect cell viability in each group of cells, C1 1-BODIPY probe method was used to detect intracellular lipid ROS levels in each group of cells, and PGSK probe method was used to detect iron content in cells.

The experimental data were expressed in means ± SD, and statistical differences between groups were determined using one-way ANOVA and Tukey's test, with p-values less than 0.05 considered significant.

Experimental results:

**Table 16 Effect of compound 1, compound 3 and compound 5 with different concentrations on cell viability of Erastin-induced neuronal cells**

| | Compound 3 (%) | Compound 5 (%) | Compound 1 (%) |
|---|---|---|---|
| Control group | 100 ± 8.4 | 100 ± 9.66 | 100 ± 6.18 |
| Model control group | 48.24 ± 5.73*** | 50.37 ± 4.9*** | 49.85 ± 4.14*** |
| 1 μM | 52.87 ± 2.55 | 58.95 ± 7.21 | 57.01 ± 2.82 |
| 2.5 μM | 56.6 ± 4.76 | 62.63 ± 7.82 | 61.77 ± 6.56 |
| 5 μM | 62.52 ± 8.51 | 67.38 ± 6.73 | 65.4 ± 5.81 |
| 10 μM | 66.77 ± 6.27 | 73.74±7.33# | 76.62±5.99## |
| 20 μM | 73.21±8.1# | 79.85±6.34## | 80.62±5.39## |
| 40 μM | 77.95±6.34## | 84.2±4.71## | 83.43±6.21## |

After incubation, the CCK8 experiment was used to detect the cell viability of each group of cells. The results were presented as Mean ± SD. Compared with control group, *** p<0.0001. Compared with model control group, #P<0.05, ##P<0.01.

As can be seen from Table 16, the cell viability of the model group was significantly reduced compared with the

control group ($p<0.0001$); when the concentration of compound 3 is greater than 10 μM, the cell viability was significantly increased compared with the model group ($p<0.05$); and when the concentrations of compound 5 and compound 1 are greater than 5 μM, the cell viability was significantly increased compared with the model group ($p<0.05$).

**Table 17 Effect of compound 1, compound 3 and compound 5 with different concentrations on lipid ROS levels in Erastin-induced nerve cells**

| | Compound 3(%) | Compound 5 (%) | Compound 1 (%) |
|---|---|---|---|
| Control group | 100 ± 10.41 | 100 ± 8.24 | 100 ± 10.71 |
| Model control group | 151.96 ± 5.14*** | 152.74 ± 3.88*** | 157.42 ± 5.12*** |
| 1 μM | 146.56 ± 7.67 | 145.89 ± 5.33 | 150.18 ± 6.05 |
| 2.5 μM | 141.34 ± 4.22 | 141.47 ± 6.53 | 142.58 ± 3.01 |
| 5 μM | 136.81 ± 7.9 | 133.09 ± 9.69 | 137.9 ± 6.72 |
| 10 μM | 132.38 ± 2.23 | 128.99±9.84# | 132.33±6.57## |
| 20 μM | 125.33±9.79# | 122.71±8.5## | 121.82±5.9## |
| 40 μM | 120.37±10.16## | 114.82±7.13## | 114.13±8.82## |

After incubation, the C11-BODIPY probe method was used to detect the lipid ROS levels in each group of cells. The results were presented as Mean ± SD. Compared with the control group, ***$p< 0.0001$; compared with the model group, #$p< 0.05$, ##$p< 0.01$.

As can be seen from Table 17, the intracellular lipid ROS levels in the model group increased significantly compared with the control group ($p<0.0001$); when the concentration of compound 3 is greater than 10 μM, the intracellular lipid ROS levels decreased significantly compared with the model group ($p<0.05$); and when the concentrations of compound 5 and compound 3 are greater than 5 μM, the intracellular lipid ROS levels decreased significantly compared with the model control group ($p<0.01$).

**Table 18 Effect of compound 1, compound 3 and compound 5 with different concentrations on iron content in Erastin-induced neuronal cells**

| | Compound 3 | Compound 5 | Compound 1 |
|---|---|---|---|
| Control group | 100 ± 9.28 | 100 ± 9.79 | 100 ± 10.88 |
| Model control group | 49 ± 5.12*** | 50.83 ± 7.37*** | 51.49 ± 4.73*** |
| 1 μM | 56.48 ± 3.32 | 56.75 ± 9.31 | 61.06 ± 7.12 |
| 2.5 μM | 62.3 ± 7 | 68.32 ± 6.01 | 66.81 ± 7.21 |
| 5 μM | 67.06 ± 11.62 | 72.65 ± 7 | 71.85 ± 4.47 |
| 10 μM | 71.61 ± 9.86 | 76.52±7.86# | 77.52±12.88# |
| 20 μM | 76.67±7.25# | 80.28±6.96## | 82.16±6.86## |
| 40 μM | 83.81±9.91## | 87.37±9.57## | 86.69±8.01## |

After incubation, the PGSK probe method was used to detect the intracellular iron content. The results were presented as Mean ± SD. Compared with the control group, *** $p<0.0001$; Compared with the model group, #$p<0.05$, ##$p<0.01$.

As can be seen from Table 18, compared with the control group ($p<0.0001$), the fluorescence intensity of intracellular PGSK probe in the model group was significantly weakened and the iron content was significantly elevated ($p<0.0001$); when the concentration of compound 3 is greater than 10 μM, the intracellular iron content was significantly decreased compared with the model group ($p<0.05$); when the concentrations of compound 5 and compound 3 are greater than 5 μM, the intracellular iron content was significantly decreased compared with the model group ($p< 0.05$).

**Table 19 Effect of Compound 1, Compound 3 and Compound 5 with different concentrations on the expression of GPX4 mRNA in Erastin-induced neuronal cells**

| | Compound 3 | Compound 5 | Compound 1 |
|---|---|---|---|
| Control group | 1.0016 ± 0.07 | 1.0031 ± 0.1 | 1.0029 ± 0.09 |
| Model control group | 0.4008 ± 0.04*** | 0.3796 ± 0.03*** | 0.3804 ± 0.04*** |
| 1 μM | 0.4359 ± 0.05 | 0.4777 ± 0.07 | 0.4845 ± 0.06 |
| 2.5 μM | 0.4875 ± 0.01 | 0.5373 ± 0.08 | 0.5759 ± 0.07 |
| 5 μM | 0.5407 ± 0.06 | 0.6033±0.06# | 0.6469±0.08# |
| 10 μM | 0.6152±0.07## | 0.6942±0.01## | 0.7093±0.15## |
| 20 μM | 0.6765±0.07## | 0.7693±0.1## | 0.7664±0.08## |
| 40 μM | 0.7421±0.08## | 0.8368±0.07## | 0.8503±0.06## |

After incubation, real time PCR was used to detect the mRNA expression of GPX4 in each group of cells. The results were presented as Mean ± SD. Compared with the control group, *"p<0.0001; Compared with the model group, # p<0.05, # p<0.01.

As can be seen from Table 19, GPX4 mRNA expression in cells of the model group was significantly decreased compared with the control group (p<0.0001); when the concentration of compound 3 is greater than 5 μM, the cellular GPX4 mRNA expression level was significantly elevated compared with the model group (p<0.05); and when the concentrations of compound 5 and compound 3 are greater than 2.5 μM, the cellular GPX4 mRNA expression level was significantly increased compared with the model group (p<0.01).

**Conclusion:** Compound 1, Compound 3 and Compound 5 all significantly increased Erastin-induced rat neuronal cell viability and GPX4 mRNA expression, and significantly decreased intracellular lipid ROS levels and iron content. The above findings indicated that Compound 1, Compound 3 and Compound 5 all inhibited Erastin-induced ferroptosis in rat neuronal cells.

**Example 9 Evaluation of regressive effect of Compound 1, Compound 3, and Compound 5 on vascular inflammation**

**Experimental method:** 360 tails of 5 dpf macrophage fluorescent zebrafish were randomly selected and placed in beakers with 30 tails in each beaker, compound (1), compound (3), and compound (5) were given intravenously, and the positive control group was given atorvastatin calcium by dissolving it in water at a concentration of 30 μg/mL, while the normal control group (fish farming water-treated zebrafish) and the model control group were set up, and the volume of each beaker (experimental group) was 25 mL. Except for the normal control group, all other groups were given high-lipids and high-sucrose feed by dissolving it in water. Compound (1), compound (3) and compound (5) group were co-treated with high-lipids and high-sucrose feed for 30 h. The treatment was carried out up to 9 dpf, and 10 zebrafish were randomly selected from each experimental group to collect the intensity of the macrophage fluorescence within the tail vein and around the tail vein of the zebrafish under fluorescence microscope, and the results of the statistical analysis were used to evaluate the regressive effect of compound (1), compound (3), compound (5) on zebrafish vascular inflammation in the high sugar and high fat model.

$$\text{Anti-inflammatory effect (\%)} = \frac{[\text{S(model control group)} - \text{S(test product group)}]}{\text{S(model control group)}} \times 100\%$$

**Experimental results:**

**Table 20 Regressive effect of the test product on vascular inflammation in high-glucose and high-fat zebrafish (n=10)**

| Group | Dosage (ng/tail) | Fluorescence intensity of perivascular macrophage (pixels, Mean ± SE) | Anti-inflammatory effect (%) |
|---|---|---|---|
| Normal control group | | 116966 ± 7407 | - |
| Model control group | - | 184631 ± 3800 | - |
| Atorvastatin calcium | 30 μg/mL | 106352 ± 6663 | 42*** |
| Compound (3) | 11.1 | 113809 ± 7995*** | 38*** |
| | 33.3 | 118747 ± 6434*** | 36*** |
| | 100 | 118504 ± 10539*** | 36*** |
| Compound (5) | 27.8 | 113678 ± 4811*** | 38*** |
| | 83.3 | 118039 ± 9198*** | 36*** |
| | 250 | 115627 ± 5934*** | 37*** |
| Compound (1) | 222 | 134308 ± 6117*** | 27*** |
| | 667 | 116848 ± 5036*** | 37*** |
| | 2000 | 108154 ± 4723*** | 41*** |
| Compared with model control group, *** p<0.001. | | | |

**Conclusion:** Compound (1), compound (3) and compound (5) showed significant elimination effect on vascular inflammation in high-glucose and high-fat zebrafish, under the dosage conditions of this experiment.

**Example 10 Preparation of injection**

**Table 21 Formula table for preparing injection of compound (1)**

| Prescription | Prescription dosage |
|---|---|
| Compound (1) | 1000 mg |
| Glycerol | 10 g |
| Sodium chloride | 3.5 g |
| Sodium citrate dihydrate | 150 mg |
| 0.1M hydrochloric acid or sodium hydroxide | Appropriate amount |
| Injection water was added to | 500 ml |
| pH range of solution | 5.0-6.5 |

80% of the total amount of injection water was taken and added with the prescribed amount of sodium chloride, sodium citrate dihydrate, glycerol, and compound (1) in sequence. The mixture was stirred to dissolve, and adjusted to pH 5.0-6.5 with 0.1M hydrochloric acid or sodium hydroxide. The product was obtained by adding injection water to the volume. The resulting solution was subpackaged into 10 ml glass vials and sterilized under heat and pressure to obtain the compound injection. Each vial contains 20 mg of compound (1).

**Example 11 Preparation of lyophilized powder for injection**

**Table 22 Formula table for preparing lyophilized powder for injection of compound (1)**

| Prescription | Prescription dosage | Function |
|---|---|---|
| Compound (1) | 0.1~0.5g | Active Ingredients |
| Sodium hydroxide or hydrochloric acid | Appropriate amount | PH regulator |
| Water for injection | Add to 2mL | Solvent |

A prescribed amount of compound (1) was added into 80% of the prescribed amount of water for injection, and stirred to dissolve; the mixture was added with water for injection to the prescribed amount. The above solution was added with activated carbon (0.1-0.5%) and stirred for dozens of minutes. After decolorization by activated carbon and filtration to remove the activated carbon, the resulting solution was added with sodium hydroxide solution (or hydrochloric acid solution) to adjust pH value. Samples were taken for semi-finished product testing. After secondary filtration through a 0.22 μm filter, bottle-filling was performed. Performing lyophilization according to the set lyophilization parameters, plugging, capping, and packaging.

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A use of a compound of formula I or a pharmaceutically acceptable salt thereof in the preparation of a pharmaceutical composition or formulation, the pharmaceutical composition or formulation comprises the compound of formula I as active ingredient, and the pharmaceutical composition or formulation is used for:

   (a) prevention and/or treatment of thrombosis-related diseases;
   (b) anti-inflammatory treatment; and/or
   (c) treatment of neuronal damage caused by cerebral infarction, traumatic brain injury, cerebral hemorrhage, or brain tumor surgery;

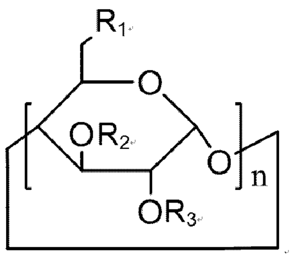

(I)

wherein,
$R_1$ is selected from the group consisting of: hydroxyl, C1-C6 alkoxy, halogen, substituted or unsubstituted -O-C1-C6 alkyl, and

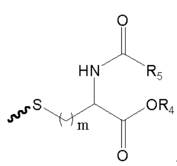

,

, wherein the substituted refers to substitution with sulfonic acid group or hydroxyl;
wherein, m is an integer selected from 1 to 6;
the carbon atom of

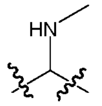 in 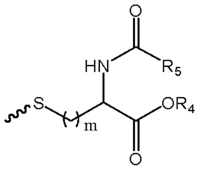

is a chiral carbon atom, and the chirality is selected form the group consisting of:

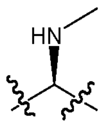 and 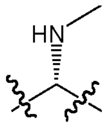;

$R_4$ is a metal ion selected from the group consisting of: $Na^+$, $K^+$, $Li^+$, and $Cs^+$; $R_5$ is C1-C6 alkyl, C3-C8 cycloalkyl, C2-C6 alkenyl, C2-C6 alkynyl, aryl or heteroaryl;
$R_2$ and $R_3$ are each independently H, C1-C6 alkyl, C2-C6 alkenyl, C2-C6 alkynyl, C3-C8 cycloalkyl, C2-C6 hydroxyalkyl, or -(C1-C3 alkylene)-COOH;
n is an integer selected from 6 to 12.

**2.** The use according to claim 1, wherein the thrombosis-related disease is selected from the group consisting of: cerebral thrombosis, cerebral infarction (acute ischemic stroke) and its resulting neuronal injury in nervous tissue, cerebral edema, myocardial infarction, pulmonary embolism, or a combination thereof.

**3.** The use according to claim 1, wherein the pharmaceutical composition or formulation is used for preventing and/or treating thrombosis-related diseases.

**4.** The use according to claim 1, wherein the pharmaceutical composition or formulation is used for treating neuronal damage caused by cerebral infarction, traumatic brain injury, cerebral hemorrhage, or brain tumor surgery.

**5.** The use according to claim 1, wherein the dosage form of the pharmaceutical composition or formulation is selected from the group consisting of: injection, lyophilized powders for injection, sustained-release, controlled release, enteric-coated tablets or capsules, granules.

**6.** A use of a combination of the compound of formula I and urokinase for the preparation of a medication for treating thrombosis-related diseases.

**7.** A use of a combination of the compound of formula I and butylphthalide for the preparation of a medication for treating neurological damages caused by cerebral infarction, traumatic brain injury, cerebral hemorrhage, or brain tumor surgery.

**8.** A pharmaceutical composition or formulation, wherein comprising (a) active ingredient, the active ingredient includes the compound of formula I, or a pharmaceutically acceptable salt thereof; and (b) pharmaceutically acceptable carriers, the pharmaceutical composition or formulation is used for:

(a) prevention and/or treatment of thrombosis-related diseases;
(b) anti-inflammatory treatment; and/or
(c) treatment of neuronal damages caused by cerebral infarction, traumatic brain injury, cerebral hemorrhage, or brain tumor surgery.

**9.** A kit, wherein the kit comprising:

(1) a first container, and a first pharmaceutical composition in the first container, the first pharmaceutical composition comprises a first compound, or a pharmaceutically acceptable salt thereof, and pharmaceutically acceptable carriers;
(2) a $n^{th}$ container, and a $n^{th}$ pharmaceutical composition in the $n^{th}$ container, the $n^{th}$ pharmaceutical composition comprises a $n^{th}$ compound, or a pharmaceutically acceptable salt thereof, and pharmaceutically acceptable

carriers; wherein, n is an positive integer selected from 2 to 8;

wherein, both the first compound and the $n^{th}$ compound are compounds of formula I or the pharmaceutically acceptable salts thereof, or the first compound is a compound of formula I or the pharmaceutically acceptable salt thereof, and at least one of the $n^{th}$ compound is an additional active substance, wherein the compound of formula I is as defined in claim 1;
and/or (3) an optional instruction manual.

**10.** A method for treating diseases, the disease is as described in claim 1, wherein the method comprising a step of: administering the compound of formula I or a pharmaceutically acceptable salt thereof to a subject in need thereof, wherein the compound of formula I is as described in claim 1.

## INTERNATIONAL SEARCH REPORT

International application No.
**PCT/CN2021/143944**

**A. CLASSIFICATION OF SUBJECT MATTER**

A61K 31/724(2006.01)i; C12N 9/72(2006.01)i; A61K 31/365(2006.01)i; A61K 9/08(2006.01)i; A61K 9/16(2006.01)i; A61K 9/19(2006.01)i; A61K 9/28(2006.01)i; A61K 9/48(2006.01)i; A61K 9/00(2006.01)i; A61P 7/02(2006.01)i; A61P 9/10(2006.01)i; A61P 43/00(2006.01)i

According to International Patent Classification (IPC) or to both national classification and IPC

**B. FIELDS SEARCHED**

Minimum documentation searched (classification system followed by classification symbols)

A61K; A61P; C12N

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, DWPI, VEN, CJFD, CNKI, ISI WEB OF SCIENCE, REGISTRY, CAPLUS: 环糊精, Cyclodextrin, 栓塞, 梗塞, 血栓, 心梗, 卒中, 中风, 脑梗, 溶栓, 抗血小板, 抗凝, 神经元损伤, 神经保护, 血管炎, 炎症, Thrombotic, embolism, infarction, thrombosis, stroke, thrombolysis, antiplatelet, anticoagulants, neuronal damage, neuroprotection, vasculitis, inflammation, 尿激酶, Urokinase, 丁苯酞, Butylphthalide, STN structural formula search etc.

**C. DOCUMENTS CONSIDERED TO BE RELEVANT**

| Category* | Citation of document, with indication, where appropriate, of the relevant passages | Relevant to claim No. |
|---|---|---|
| X | 张严 等 (ZHANG, Yan et al.). "beta-环糊精及衍生物作为生物活性分子研究进展 (Research Progress of β-Cyclodextrins and Its Derivatives as Active Pharmaceutical Ingredients)." *药学研究 (Journal of Pharmaceutical Research),* Vol. 37, No. (10), 31 December 2018 (2018-12-31), Pages 589-592 and 611, in particular sections 1, 3 and 4 | 1-9 |
| X | WO 0004888 A2 (CEREBRUS PHARM LTD. et al.) 03 February 2000 (2000-02-03) Entire document, in particular claims 1-16, description, page 4, line 6 to page 11 | 1-3, 5, 6, 8 and 9 (in part), 4 and 7 |
| X | BECKTEL, D.A. et al. "Repeated Administration of 2-Hydroxypropyl- Beta -Cyclodextrin (HP- beta-CD) Attenuates the Chronic Inflammatory Response to Experimental Stroke." *J Neurosci.,* Vol. 42, No. (2), 24 November 2021 (2021-11-24), Pages 325-348, in particular abstract, page 329, left-hand column, paragraph 4 | 1-3, 5, 6, 8 and 9 (in part), 4 and 7 |
| X | NAGASE, Y. et al. "Inhibitory Effect of Sulfobutyl Ether Beta-Cyclodextrin on DY-9760e-Induced Cellular Damage: in Vitro and in Vivo Studies." *J Pharm Sci.,* Vol. 92, No. (12), 31 December 2003 (2003-12-31), pp. 2466-2474 | 1-3, 5, 6, 8 and 9 (in part) |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 September 2022** | **10 October 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2021/143944**

**C. DOCUMENTS CONSIDERED TO BE RELEVANT**

| Category* | Citation of document, with indication, where appropriate, of the relevant passages | Relevant to claim No. |
|---|---|---|
| X | JP 2004323443 A (UNIV TOKAI) 18 November 2004 (2004-11-18)<br>Entire document, in particular claims 1-4, description, paragraphs [0001] and [0010]-[0023] | 1-3, 5, 6, 8 and 9 (in part) |
| X | US 2020353009 A1 (KYUNGPOOK NAT UNIV IND ACADEMIC COOP FOUND) 12 November 2020 (2020-11-12)<br>description, embodiments 3 and 4 | 1-3, 5, 6, 8 and 9 (in part), 4 and 7 |
| X | KRISANOVA, N. et al. "Neuroprotection by Lowering Cholesterol: A decrease in Membrane Cholesterol Content Reduces Transporter-Mediated Glutamate Release from Brain Nerve Terminals."<br>*Biochimica et Biophysica Acta,* Vol. 1822, No. (10), 17 June 2012 (2012-06-17), pp. 1553-1561 | 1-3, 5, 6, 8 and 9 (in part), 4 and 7 |
| A | CN 102060941 A (QI, Youmao) 18 May 2011 (2011-05-18)<br>Entire document, in particular the claims | 1-9 |

Form PCT/ISA/210 (second sheet) (January 2015)

**INTERNATIONAL SEARCH REPORT**

International application No.
**PCT/CN2021/143944**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **10**
because they relate to subject matter not required to be searched by this Authority, namely:

[1] A method for treating a human or animal body (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

[1] The first group: claims 1-3, 5, 6, 8 and 9 (in part) set forth the use of a compound of formula I, a pharmaceutical composition or a preparation and a kit, wherein the use thereof is to prevent and/or treat thrombus-related diseases, and the thrombus-related diseases exclude neuronal damage in nerve tissue caused by cerebral infarction (acute ischemic stroke);

[2] the second group: claims 1, 5, 8 and 9 (in part) set forth the use of a compound of formula I, a pharmaceutical composition or a preparation and a kit, wherein the use thereof is anti-inflammatory treatment; and

[3] the third group: claims 1-3, 5, 6, 8 and 9 (in part), and claims 4 and 7 set forth the use of a compound of formula I, a pharmaceutical composition or a preparation and a kit, wherein the use thereof is to treat neuronal damage caused by cerebral infarction, traumatic brain injury, cerebral hemorrhage or brain tumor surgery.

[4] The above three groups of claims respectively relate to different uses of the compound of formula I. The compound is a known compound. Therefore, the three groups of claims do not have the same or corresponding special technical features, do not have any technical relationship therebetween, do not meet the requirement of unity of invention, and thus do not comply with PCT Rule 13.1.

Form PCT/ISA/210 (continuation of first sheet) (January 2015)

**INTERNATIONAL SEARCH REPORT**

International application No.
**PCT/CN2021/143944**

**Box No. III Observations where unity of invention is lacking (Continuation of item 3 of first sheet)**

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

Form PCT/ISA/210 (continuation of first sheet) (January 2015)

**INTERNATIONAL SEARCH REPORT**
**Information on patent family members**

International application No.
**PCT/CN2021/143944**

| Patent document cited in search report | | | Publication date (day/month/year) | Patent family member(s) | | | Publication date (day/month/year) |
|---|---|---|---|---|---|---|---|
| WO | 0004888 | A2 | 03 February 2000 | GB | 9815785 | D0 | 16 September 1998 |
| | | | | AU | 5050899 | A | 14 February 2000 |
| JP | 2004323443 | A | 18 November 2004 | None | | | |
| US | 2020353009 | A1 | 12 November 2020 | KR | 101906578 | B1 | 10 October 2018 |
| | | | | WO | 2019022396 | A1 | 31 January 2019 |
| CN | 102060941 | A | 18 May 2011 | EP | 2644624 | A1 | 02 October 2013 |
| | | | | DK | 2644624 | T3 | 01 May 2017 |
| | | | | WO | 2012068981 | A1 | 31 May 2012 |
| | | | | JP | 2013543915 | A | 09 December 2013 |
| | | | | CA | 2819007 | A1 | 31 May 2012 |
| | | | | US | 2013244979 | A1 | 19 September 2013 |
| | | | | US | 2019010255 | A1 | 10 January 2019 |
| | | | | ES | 2622332 | T3 | 06 July 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **J. SAMBROOK et al.** Molecular Cloning: A Laboratory Manual. Science Press, 1989
- *CHEMICAL ABSTRACTS*, 1309580-41-3